(19) Europäisches Patentamt
European Patent Office
Office européen des brevets

(11) EP 4 717 774 A1

(12) EUROPEAN PATENT APPLICATION
published in accordance with Art. 153(4) EPC

(43) Date of publication:
**01.04.2026 Bulletin 2026/14**

(21) Application number: **24811109.8**

(22) Date of filing: **21.05.2024**

(51) International Patent Classification (IPC):
*C12N 15/31* (2006.01) *C12N 1/15* (2006.01)
*C12N 1/19* (2006.01) *C12N 1/21* (2006.01)
*C12P 17/12* (2006.01)

(52) Cooperative Patent Classification (CPC):
**C07K 14/195; C12N 15/74; C12N 15/80; C12N 15/81; C12P 17/12**

(86) International application number:
**PCT/JP2024/018666**

(87) International publication number:
**WO 2024/242102 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.05.2023 JP 2023084745**

(71) Applicants:
- **Mitsui Chemicals Crop & Life Solutions, Inc.**
  **Tokyo 103-0027 (JP)**
- **National Institute of Advanced Industrial Science and Technology**
  **Chiyoda-ku**
  **Tokyo 100-8921 (JP)**

(72) Inventors:
- **FUKUDA TAKEUCHI Haruka**
  **Tokyo 103-0027 (JP)**
- **YAMAMOTO Kentaro**
  **Mobara-shi, Chiba 297-0017 (JP)**
- **TAMANO Koichi**
  **Tsukuba-shi, Ibaraki 305-8560 (JP)**
- **TAMURA Tomohiro**
  **Tsukuba-shi, Ibaraki 305-8560 (JP)**
- **ABURATANI Sachiyo**
  **Tsukuba-shi, Ibaraki 305-8560 (JP)**

(74) Representative: **Dolphin, Kirsty Mairi et al**
**Venner Shipley LLP**
**200 Aldersgate**
**London EC1A 4HD (GB)**

Remarks:
The complete document including Reference Table(s) and the Sequence Listing(s) can be downloaded from the EPO website

# (54) METHOD FOR PRODUCING PF1378A, PROTEIN, NUCLEIC ACID, AND TRANSFORMANT

(57) A production method for PF1378A, the method including a step of bringing one or more proteins selected from the group consisting of (1) a protein consisting of an amino acid sequence set forth in SEQ ID NO: 2, (2) a protein that consists of an amino acid sequence having a sequence identity of 70% or more with respect to the amino acid sequence set forth in SEQ ID NO: 2 and has an activity of converting PF1378B to PF1378A, (3) a protein encoded by a base sequence set forth in SEQ ID NO: 1, and (4) a protein that is encoded by a base sequence having a sequence identity of 70% or more with respect to the base sequence set forth in SEQ ID NO: 1 and has an activity of converting PF1378B to PF1378A, into contact with PF1378B, thereby obtaining PF1378A.

EP 4 717 774 A1

## Description

TECHNICAL FIELD

The present invention relates to a production method for PF1378A. More specifically, the present invention relates to a production method for PF1378A, a protein, a nucleic acid, and a transformant. Priority is claimed on Japanese Patent Application No. 2023-084745, filed on May 23, 2023, the content of which is incorporated herein by reference.

BACKGROUND ART

PF1378A (16-Keto-aspergillimide, CAS number: 199784-50-4) is a compound represented by Formula (1).

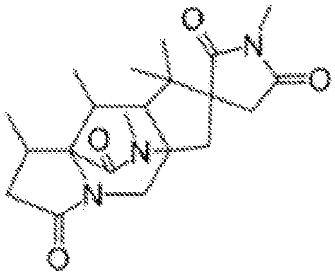

・・・(1)

PF1378A has been reported as a metabolite produced by a filamentous fungus of the genus Aspergillus, and is known as an anthelmintically active substance against third instar larvae of Haemonchus contortus, which is a gastrointestinal parasitic nematode of mammals (see, for example, Non-Patent Document 1). Furthermore, Patent Document 1 and 2 describes that PF1378A exhibits an insecticidal activity against agricultural pests such as Nilaparvata lugens. In addition, Patent Document 3 describes that PF1378A exhibits an insecticidal activity against animal parasitic pests such as ticks and fleas, and has safety against mammals. In the related art, production technology for PF1378A by liquid culture is not known.

Citation List

Patent Documents

Patent Document 1: PCT International Publication No. WO2008/156165
Patent Document 2: PCT International Publication No. WO2010/071218
Patent Document 3: PCT International Publication No. WO2010/071219

Non-Patent Document

Non-Patent Document 1: Banks R. M., et al., Novel anthelmintic metabolites from an Aspergillus species; the aspergillimides, The Journal of Antibiotics, 50 (10), 840-846, 1997.

SUMMARY OF INVENTION

Technical Problem

As described above, PF1378A is a useful compound, but it is difficult to chemically synthesize PF1378A and it is necessary to biosynthesize PF1378A by microbial culture or the like. Therefore, it is important to elucidate a biosynthetic pathway of PF1378A and apply it to production of PF1378A. An object of the present invention is to provide production technology for PF1378A.

Solution to Problem

The present inventors have demonstrated that in a final reaction of biosynthesis of PF1378A by a filamentous fungus of the genus Aspergillus, a protein encoded by a g5141 gene converts PF1378B (asperparaline A, CAS number:

195966-93-9) to PF1378A, thereby completing the present invention. The g5141 gene and a protein encoded by the g5141 gene are each a novel gene and a novel protein, which have been discovered this time by the present inventors. PF1378B is a compound represented by Formula (2).

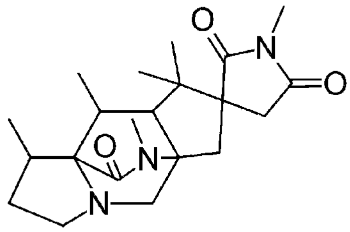

・・・(2)

The present inventors have further demonstrated that in the biosynthesis of PF1378B by the filamentous fungus of the genus Aspergillus, a protein encoded by a g5151a gene converts PF1378C (asperparaline B, CAS number: 227598-73-4) to PF1378B. The g5151a gene and a protein encoded by the g5151a gene are each a novel gene and a novel protein, which have been discovered this time by the present inventors. PF1378C is a compound represented by Formula (3).

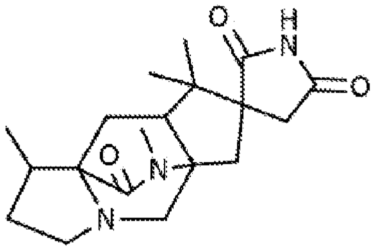

・・・(3)

The present invention includes the following aspects.

[1] A production method for PF1378A, the method including:
a step (a) of bringing one or more proteins selected from the group consisting of the following (1) to (4) into contact with PF1378B to obtain PF1378A,

(1) a protein consisting of an amino acid sequence set forth in SEQ ID NO: 2,
(2) a protein consisting of an amino acid sequence having a sequence identity of 70% or more with respect to the amino acid sequence set forth in SEQ ID NO: 2 and has an activity of converting PF1378B to PF1378A,
(3) a protein encoded by a base sequence set forth in SEQ ID NO: 1, and
(4) a protein encoded by a base sequence having a sequence identity of 70% or more with respect to the base sequence set forth in SEQ ID NO: 1 and has an activity of converting PF1378B to PF1378A.

[2] The production method according to [1],
in which the step (a) is carried out in a cell of a microorganism.
[3] The production method according to [1] or [2], further including, before the step (a):

a step (b) of bringing one or more proteins selected from the group consisting of the following (5) to (8) into contact with PF1378C to obtain PF1378B,
(5) a protein consisting of an amino acid sequence set forth in SEQ ID NO: 7,
(6) a protein consisting of an amino acid sequence having a sequence identity of 70% or more with respect to the amino acid sequence set forth in SEQ ID NO: 7 and has an activity of converting PF1378C to PF1378B,
(7) a protein encoded by a base sequence set forth in SEQ ID NO: 6, and
(8) a protein encoded by a base sequence having a sequence identity of 70% or more with respect to the base sequence set forth in SEQ ID NO: 6 and has an activity of converting PF1378C to PF1378B.

[4] The production method according to [3],
in which the step (b) is carried out in a cell of a microorganism.
[5] A protein consisting of an amino acid sequence set forth in SEQ ID NO: 2, or consisting of an amino acid sequence having a sequence identity of 70% or more with respect to the amino acid sequence set forth in SEQ ID NO: 2 and has

an activity of converting PF1378B to PF1378A.
[6] A protein consisting of an amino acid sequence set forth in SEQ ID NO: 7, or consisting of an amino acid sequence having a sequence identity of 70% or more with respect to the amino acid sequence set forth in SEQ ID NO: 7 and has an activity of converting PF1378C to PF1378B.
[7] A nucleic acid encoding the protein according to [5].
[8] The nucleic acid according to [7],
in which the nucleic acid consists of a base sequence set forth in SEQ ID NO: 1; or consists of a base sequence having a sequence identity of 70% or more with respect to the base sequence set forth in SEQ ID NO: 1 and encodes a protein having an activity of converting PF1378B to PF1378A.
[9] A nucleic acid encoding the protein according to [6].
[10] The nucleic acid according to [9],
in which the nucleic acid consists of a base sequence set forth in SEQ ID NO: 6; or consists of a base sequence having a sequence identity of 70% or more with respect to the base sequence set forth in SEQ ID NO: 6 and encodes a protein having an activity of converting PF1378C to PF1378B.
[11] A transformant into which the nucleic acid according to [7] or [8] has been introduced.
[12] A transformant into which the nucleic acid according to [9] or [10] has been introduced.

It can also be said that the present invention includes the following aspects.

[P1] A production method for PF1378A, the method including:

a step of bringing a protein encoded by a g5141 gene or a mutant gene of the g5141 gene into contact with PF1378B to obtain PF1378A,
in which a cDNA of the g5141 gene has a base sequence set forth in SEQ ID NO: 1, a cDNA of the mutant gene of the g5141 gene has a base sequence having a sequence identity of 70% or more with respect to the base sequence set forth in SEQ ID NO: 1, and
the protein that is encoded by the mutant gene of the g5141 gene has an activity of converting PF1378B to PF1378A.

[P2] The production method according to [P1],
in which the step is carried out in a cell of a microorganism.
[P3] A nucleic acid encoding a g5141 gene or a mutant gene of the g5141 gene,
in which a cDNA of the g5141 gene has a base sequence set forth in SEQ ID NO: 1, a cDNA of the mutant gene has a base sequence having 70% or more sequence identity with respect to the base sequence set forth in SEQ ID NO: 1, and a protein encoded by the mutant gene has an activity of converting PF1378B to PF1378A.
[P4] A transformant into which the nucleic acid according to [P3] has been introduced.

Advantageous Effects of Invention

According to the present invention, it is possible to provide production technology for PF1378A.

DESCRIPTION OF EMBODIMENTS

[Production Method for PF1378A]

In one embodiment, the present invention provides a production method for PF1378A, the method including a step (a) of bringing one or more proteins selected from the group consisting of the following (1) to (4) into contact with PF1378B to obtain PF1378A,

(1) a protein consisting of an amino acid sequence set forth in SEQ ID NO: 2,
(2) a protein consisting of an amino acid sequence having a sequence identity of 70% or more with respect to the amino acid sequence set forth in SEQ ID NO: 2 and has an activity of converting PF1378B to PF1378A,
(3) a protein encoded by a base sequence set forth in SEQ ID NO: 1, and
(4) a protein encoded by a base sequence having a sequence identity of 70% or more with respect to the base sequence set forth in SEQ ID NO: 1 and has an activity of converting PF1378B to PF1378A.

In one aspect, the production method of the present embodiment is a production method including a step (a) of bringing a protein encoded by a g5141 gene or a mutant gene thereof into contact with PF1378B to obtain PF1378A, in

which a cDNA of the g5141 gene has a base sequence set forth in SEQ ID NO: 1, a cDNA of the mutant gene has a base sequence having a sequence identity of 70% or more with respect to the base sequence set forth in SEQ ID NO: 1, and the protein encoded by the mutant gene has an activity of converting PF1378B to PF1378A.

As will be described later in Examples, the present inventors have found that a protein encoded by a g5141 gene converts PF1378B to PF1378A in a final reaction of biosynthesis of PF1378A by an Aspergillus aculeatinus PF1378 strain (hereinafter referred to as "A. aculeatinus PF1378" in some cases). Therefore, PF1378A can be produced by bringing a protein encoded by the g5141 gene or a mutant gene thereof into contact with PF1378B. The base sequence of a cDNA of the g5141 gene derived from A. aculeatinus PF1378 is shown in SEQ ID NO: 1. Furthermore, the amino acid sequence of the protein encoded by the g5141 gene is shown in SEQ ID NO: 2. In addition, the base sequence of a genomic DNA of the g5141 gene is shown in SEQ ID NO: 3.

In the production method of the present embodiment, the protein consisting of the amino acid sequence set forth in SEQ ID NO: 2 may include a mutation as long as it can convert PF1378B to PF1378A. Examples of the mutation include deletion, insertion, substitution, and addition of one or several amino acids. Here, the expression of one or several encompasses the numbers of 1 to 150, 1 to 100, 1 to 50, 1 to 30, 1 to 20, 1 to 10, 1 to 5, 1 to 3, and the like.

Examples of the mutant protein of the amino acid sequence set forth in SEQ ID NO: 2 include a mutant protein that consists of an amino acid sequence having a sequence identity of 70% or more, 80% or more, 90% or more, 95% or more, 96% or more, 97% or more, 98% or more, or 99% or more with respect to the amino acid sequence set forth in SEQ ID NO: 2 and has an activity of converting PF1378B to PF1378A.

The sequence identity of the amino acid sequence can be calculated by a method known to those skilled in the art, but can be calculated using, for example, a Blastp (protein-protein BLAST) which is a search program of a basic local alignment search tool (BLAST).

Alternatively, the sequence identity of a target amino acid sequence with respect to a reference amino acid sequence can be determined as follows, for example. First, the reference amino acid sequence and the target amino acid sequence are aligned. Here, each amino acid sequence may include gaps to maximize the sequence identity. Subsequently, the number of matching amino acids in the reference amino acid sequence and the target amino acid sequence is calculated, and the sequence identity can be determined according to Expression (F2).

$$\text{Sequence identity (\%)} = \text{Number of matching amino acids/Total number of amino acids in target amino acid sequence} \times 100 \qquad \text{(F2)}$$

The g5141 gene may include a mutation as long as it can convert PF1378B to PF1378A. Examples of the mutation include deletion, insertion, substitution, and addition of one or several bases. Here, the expression of one or several encompasses the numbers of 1 to 500, 1 to 400, 1 to 300, 1 to 200, 1 to 100, 1 to 50, 1 to 30, 1 to 20, 1 to 10, 1 to 5, 1 to 3, and the like. Examples of the mutant gene of the g5141 gene include a mutant gene in which a cDNA base sequence of the mutant gene has a base sequence having a sequence identity of 70% or more, 80% or more, 90% or more, 95% or more, 96% or more, 97% or more, 98% or more, or 99% or more with respect to the base sequence set forth in SEQ ID NO: 1, and a protein encoded by the mutant gene has an activity of converting PF1378B to PF1378A.

The sequence identity of the base sequence can be calculated by a method known to those skilled in the art, but can be calculated using, for example, a Blastn (nucleotide-nucleotide BLAST) which is a search program of a basic local alignment search tool (BLAST).

Alternatively, a sequence identity of a target base sequence with respect to a reference base sequence can be determined as follows, for example. First, the reference base sequence and the target base sequence are aligned. Here, each base sequence may include gaps to maximize the sequence identity. Subsequently, the number of matching bases in the reference base sequence and the target base sequence is calculated, and the sequence identity can be determined according to Expression (F1).

$$\text{Sequence identity (\%)} = \text{Number of matching bases/Total number of bases in target base sequence} \times 100 \qquad \text{(F1)}$$

The sequence identity of the mutant gene of the g5141 gene with respect to the g5141 gene may be low in a region other than a region that encodes an active domain. Here, the active domain refers to a domain having an activity of converting PF1378B in a protein encoded by the g5141 gene to PF1378A.

From a comparison of amino acid sequences of the g5141 gene and a homolog thereof, it was considered that the regions of the 295th to 408th bases, the 504th to 707th bases, the 1,341st to 1,637th bases, the 1,344th to 1,502nd bases, and the 1,639th to 1,779th bases in the base sequence of a genomic DNA of the g5141 gene set forth in SEQ ID No. 3 are highly conserved. From this, it is considered that the regions of the 295th to 408th bases, the 504th to 707th bases, the 1,341st to 1,637th bases, the 1,344th to 1,502nd bases, and the 1,639th to 1,779th bases in the base sequence set forth in SEQ ID NO: 3 are important for the activity of the protein encoded by the g5141 gene. Therefore, the sequence identity of

the mutant gene of the g5141 gene with respect to the g5141 gene is preferably 70% or more, 80% or more, 90% or more, 95% or more, 96% or more, 97% or more, 98% or more, or 99% or more in the regions corresponding to the 295th to 408th bases, the 504th to 707th bases, the 1,341st to 1,637th bases, the 1,344th to 1,502nd bases, and the 1,639th to 1,779th bases in SEQ ID NO: 3.

In addition, in the mutant gene of the g5141 gene, the sequence identity with respect to the g5141 gene may be less than 90% in the region other than the regions corresponding to the 295th to 408th bases, the 504th to 707th bases, the 1,341st to 1,637th bases, the 1,344th to 1,502nd bases, and the 1,639th to 1,779th bases in SEQ ID NO: 3. In this case, the lower limit of the sequence identity may be, for example, 50% or more, 60% or more, 70% or more, 75% or more, 80% or more, or 85% or more.

The mutant gene of the g5141 gene may be a homolog of the g5141 gene in an organism other than the A. aculeatinus PF1378, or may be a g5141 gene that has been artificially modified by gene recombination technology, or a homolog thereof. In the present specification, genes having similar base sequences or proteins having similar amino acid sequences are referred to as homologs.

As will be described later in Examples, the present inventors have further found that a protein encoded by a g5151a gene converts PF1378C to PF1378B in biosynthesis of PF1378B by an Aspergillus aculeatinus PF1378 strain (hereinafter referred to as an "A. aculeatinus PF1378" in some cases). Therefore, PF1378B can be produced by bringing a protein encoded by the g5151a gene or a mutant gene thereof into contact with PF1378C. The base sequence of a cDNA of the g5151a gene derived from the A. aculeatinus PF1378 is shown in SEQ ID NO: 6. Furthermore, the amino acid sequence of a protein encoded by the g5151a gene is shown in SEQ ID NO: 7. In addition, the base sequence of a genomic DNA of the g5151a gene is shown in SEQ ID NO: 8.

Therefore, the production method of the present embodiment may further include, before the step (a), a step (b) of bringing one or more proteins selected from the group consisting of the following (5) to (8) into contact to obtain PF1378B,

(5) a protein consisting of an amino acid sequence set forth in SEQ ID NO: 7,
(6) a protein consisting of an amino acid sequence having a sequence identity of 70% or more with respect to the amino acid sequence set forth in SEQ ID NO: 7 and has an activity of converting PF1378C to PF1378B,
(7) a protein encoded by a base sequence set forth in SEQ ID NO: 6, and
(8) a protein that is encoded by a base sequence having a sequence identity of 70% or more with respect to the base sequence set forth in SEQ ID NO: 6 and has an activity of converting PF1378C to PF1378B.

In one aspect, the production method of the present embodiment may be the production method further including, before the step (a), a step (b) of bringing a protein encoded by a g5151a gene or a mutant gene thereof into contact with PF1378C to obtain PF1378B, in which a cDNA of the g5151a gene has a base sequence set forth in SEQ ID NO: 6, a cDNA of the mutant gene of the g5151a gene has a base sequence having a sequence identity of 70% or more with respect to the base sequence set forth in SEQ ID NO: 1, and the protein that is encoded by the mutant gene of the g5151a gene has an activity of converting PF1378C to PF1378B.

In the production method of the present embodiment, the protein consisting of an amino acid sequence set forth in SEQ ID NO: 7 may include a mutation as long as it can convert PF1378C to PF1378B. Examples of the mutation include deletion, insertion, substitution, and addition of one or several amino acids. Here, the expression of one or several encompasses the numbers of 1 to 100, 1 to 50, 1 to 30, 1 to 20, 1 to 10, 1 to 5, 1 to 3, and the like.

Examples of the mutant protein of the amino acid sequence set forth in the SEQ ID NO: 7 include a mutant protein having an amino acid sequence having a sequence identity of 70% or more, 80% or more, 90% or more, 95% or more, 96% or more, 97% or more, 98% or more, or 99% or more with respect to the amino acid sequence set forth in the SEQ ID NO: 7 and has an activity of converting PF1378C to PF1378B. The sequence identity is the same as described above.

Similarly, the g5151a gene may include a mutation as long as it can convert PF1378C to PF1378B. Examples of the mutation include deletion, insertion, substitution, and addition of one or several bases. Here, the expression of one or several encompasses the numbers of 1 to 300, 1 to 200, 1 to 100, 1 to 50, 1 to 30, 1 to 20, 1 to 10, 1 to 5, 1 to 3, and the like. Examples of the mutant gene of the g5151a gene include a mutant gene in which a cDNA base sequence of the mutant gene has a base sequence having a sequence identity of 70% or more, 80% or more, 90% or more, 95% or more, 96% or more, 97% or more, 98% or more, or 99% or more with respect to the base sequence set forth in SEQ ID NO: 6, and a protein encoded by the mutant gene has an activity of converting PF1378C to PF1378B. The sequence identity is the same as described above.

In the production method of the present embodiment, PF1378B or PF1378C may be a natural product produced by a microorganism or may be chemically synthesized. In addition, PF1378B or PF1378C may be unpurified or purified. The purification of PF1378B or PF1378C can be carried out by a normal separation means utilizing the properties and states of PF1378B or PF1378C, for example, a solvent extraction method, an ion exchange resin method, an adsorption column chromatography, a distribution column chromatography, a gel filtration method, a dialysis method, a precipitation method, and a crystallization method, alone or in an appropriate combination thereof.

In the production method of the present embodiment, a protein encoded by the g5141 gene, the g5151a gene, or a mutant gene thereof may be purified. For the purification of the protein, affinity chromatography, hydrophobic interaction chromatography, ion exchange chromatography, gel filtration chromatography, reverse phase chromatography, and the like can be carried out alone or in an appropriate combination thereof. In addition, the protein encoded by the g5141 gene, the g5151a gene, or a mutant gene thereof may include a tag, and thus, affinity chromatography using the tag may be performed. As the tag, a known tag can be used, and examples thereof include a His tag, a FLAG tag, an MYC tag, an HA tag, and a V5 tag.

The step (a) of bringing a protein encoded by the g5141 gene or a mutant gene thereof into contact with PF1378B to obtain PF1378A may be carried out in an appropriate reaction solution. Here, examples of the reaction solution include a buffer solution that has been adjusted to an appropriate pH or salt concentration. PF1378A can be obtained by bringing the protein encoded by the g5141 gene or a mutant gene thereof into contact with PF1378B in the reaction solution.

A pH of the reaction solution can be 4.0 to 9.0. Furthermore, a concentration of PF1378B in the reaction solution can be 30 μM to 100 mM. In addition, a concentration of the protein encoded by the g5141 gene or a mutant gene thereof can be 3 nM to 100 μM. Moreover, the reaction can be carried out at 15°C to 50°C. In addition, the reaction time can be, for example, 0.5 to 400 hours.

In addition, the step (b) of bringing a protein encoded by the g5151a gene or a mutant gene thereof into contact with PF1378C to obtain PF1378B may be carried out in an appropriate reaction solution. Here, examples of the reaction solution include a buffer solution that has been adjusted to an appropriate pH or salt concentration. PF1378B can be obtained by bringing the protein encoded by the g5151a gene or a mutant gene thereof into contact with PF1378C in the reaction solution.

A pH of the reaction solution can be 4.0 to 9.0. Furthermore, a concentration of PF1378C in the reaction solution can be 30 μM to 100 mM. Furthermore, a concentration of the protein encoded by the g5151a gene or a mutant gene thereof can be 3 nM to 100 μM. Moreover, the reaction can be carried out at 15°C to 50°C. In addition, the reaction time can be, for example, 0.5 to 400 hours.

In the production method of the present embodiment, the step (a) of bringing the protein encoded by the g5141 gene or a mutant gene thereof into contact with PF1378B to obtain PF1378A may be carried out in a cell of a microorganism.

In this case, PF1378B may be synthesized in a cell of a microorganism. The biosynthesis of PF1378B and the conversion to PF1378A may be continuously carried out in the same cell of the microorganism.

In addition, the step (b) of bringing a protein encoded by the g5151a gene or a mutant gene thereof into contact with PF1378C to obtain PF1378B may be carried out in a cell of a microorganism.

In this case, PF1378C may be synthesized in a cell of a microorganism. The biosynthesis of PF1378C and the conversion to PF1378B may be continuously carried out in the same cell of the microorganism. Furthermore, the conversion of PF1378B to PF1378A may be continuously carried out in the same cell of the microorganism.

The microorganism may be an A. aculeatinus PF1378 or a microorganism other than the A. aculeatinus PF1378. Examples of the microorganism include actinomycetes, Escherichia coli, Bacillus subtilis, a yeast, and filamentous fungi.

In this case, PF1378B is synthesized in a cell of a microorganism by culturing the microorganism, and converted to PF1378A. Alternatively, PF1378C is synthesized in a cell of a microorganism, converted to PF1378B, and further converted to PF1378A.

The culture conditions for the microorganism may be appropriately selected depending on the microorganism to be used. Specific examples of the culture method include a solid culture method under an aerobic condition, a shaking culture method in a liquid medium, an aeration-stirring culture method, and a deep aerobic culture method. Examples of a temperature suitable for the culture include 15°C to 40°C. Although varying depending on a microorganism to be used, the accumulation of PF1378A reaches the highest level in 2 to 10 days under any of culture conditions. A plant cell may be used instead of the microorganism.

According to the production method of the present embodiment, a composition including PF1378A and PF1378B, or a composition including PF1378A, PF1378B, and PF1378C can be obtained. As will be described later in Examples, in the presence of a protein encoded by the g5151a gene or a mutant gene thereof, PF1378C is converted to PF1378B and thus hardly detected in some cases.

The obtained PF1378A can be purified. The purification method is the same as that for PF1378B or PF1378C described above, and for example, a solvent extraction method, an ion exchange resin method, adsorption column chromatography, distribution column chromatography, a gel filtration method, a dialysis method, a precipitation method, a crystallization method, and the like can be carried out alone or in an appropriate combination thereof.

[Protein]

In one embodiment, the present invention provides a protein consisting of the amino acid sequence set forth in SEQ ID NO: 2. The protein consisting of the amino acid sequence set forth in SEQ ID NO: 2 is a novel protein discovered by

the present inventors. As described above, PF1378A can be produced from PF1378B, using the protein of the present embodiment.

The protein of the present embodiment may be a non-natural protein, and for example, a peptide tag for purification may be added to the N-terminal or the C-terminal. The peptide tag is not particularly limited, and examples thereof include a histidine tag, a FLAG tag, and an MYC tag.

In one embodiment, the present invention provides a mutant protein that consists of an amino acid sequence having a sequence identity of 70% or more, 80% or more, 90% or more, 95% or more, 96% or more, 97% or more, 98% or more, or 99% or more with respect to the amino acid sequence set forth in SEQ ID NO: 2 and has an activity of converting PF1378B to PF1378A. As described above, PF1378A can be produced from PF1378B, using the protein of the present embodiment.

In one embodiment, the present invention provides a protein consisting of the amino acid sequence set forth in SEQ ID NO: 7. The protein consisting of the amino acid sequence set forth in SEQ ID NO: 7 is a novel protein discovered by the present inventors. As described above, PF1378B can be produced from PF1378C, using the protein of the present embodiment.

The protein of the present embodiment may be a non-natural protein, and for example, a peptide tag for purification may be added to the N-terminal or the C-terminal. The peptide tag is the same as described above.

In one embodiment, the present invention provides a mutant protein that consists of an amino acid sequence having a sequence identity of 70% or more, 80% or more, 90% or more, 95% or more, 96% or more, 97% or more, 98% or more, or 99% or more with respect to the amino acid sequence set forth in SEQ ID NO: 7 and has an activity of converting PF1378C to PF1378B. As described above, PF1378B can be produced from PF1378C, using the protein of the present embodiment.

[Nucleic Acid]

(Nucleic acid encoding g5141 gene or mutant gene thereof)

In one embodiment, the present invention provides a nucleic acid encoding a g5141 gene or a mutant gene thereof, in which a cDNA of the g5141 gene has a base sequence set forth in SEQ ID NO: 1, a cDNA of the mutant gene has a base sequence having 70% or more sequence identity with respect to the base sequence set forth in SEQ ID NO: 1, and a protein encoded by the mutant gene has an activity of converting PF1378B to PF1378A. The nucleic acid of the present embodiment may be a non-natural nucleic acid.

In the nucleic acid of the present embodiment, the g5141 gene and a mutant gene thereof are the same as described above. The mutant gene may have a base sequence having a sequence identity of 70% or more, 80% or more, 90% or more, 95% or more, 96% or more, 97% or more, 98% or more, or 99% or more with respect to the base sequence set forth in SEQ ID NO: 1, and a protein encoded by the mutant gene may have an activity of converting PF1378B to PF1378A.

The nucleic acid of the present embodiment may be in the form of a nucleic acid fragment or may be in the form of a vector. That is, the nucleic acid of the present embodiment may be a vector including a nucleic acid encoding the g5141 gene or a mutant gene thereof. The vector may be a virus, a plasmid, a phosmid, or a cosmid. More specifically, for example, in a case where the host is Escherichia coli, a λ phage-based bacteriophage or a pBR- or pUC-based plasmid may be used. In a case where the host is Bacillus subtilis, a pUB-based plasmid may be used. In a case where the host is a yeast, a YEp-, YRp-, YCp-, or YIp-based plasmid may be used.

The nucleic acid of the present embodiment preferably includes a selectable marker for selecting a transformant. Examples of the selectable marker include a drug-resistant gene and a gene complementing auxotrophy. In a case where the host is a bacterium, more specific examples of the selectable marker include an ampicillin-resistant gene, a kanamycin-resistant gene, and a tetracycline-resistant gene. Furthermore, in a case where the host is a yeast, examples thereof include a tryptophan biosynthetic gene (TRP1), an uracil biosynthetic gene (URA3), and a leucine biosynthetic gene (LEU2). Moreover, in a case where the host is a filamentous bacterium, examples thereof include a hygromycin-resistant gene, a bialaphos-resistant gene, a bleomycin-resistant gene, an aureobasidin-resistant gene, a pyrithiamine-resistant gene, and an oligomycin-resistant gene. In addition, in a case where the host is a plant, examples thereof include a kanamycin-resistant gene and a bialaphos-resistant gene.

The nucleic acid may have a transcription regulation signal or a translation regulation signal necessary for the expression of a gene. Examples of the transcription regulation signal or the translation regulation signal include a promoter, a transcription start signal, a ribosome binding site, a translation stop signal, and a transcription termination signal.

In a case where the host is Escherichia coli, examples of the promoter include a lactose operon promoter and a tryptophan operon promoter. Furthermore, in a case where the host is a yeast, examples of the promoter include an alcohol dehydrogenase gene promoter, an acid phosphatase gene promoter, a galactose assimilating gene promoter, and a glycerol-3-phosphate dehydrogenase promoter. Moreover, in a case where the host is an actinomycete, examples of the

promoter include an α-amylase gene promoter, a glucoamylase gene promoter, a translation elongation factor 1-α gene promoter, and an enolase gene promoter. In addition, in a case where the host is a plant, examples of the promoter include a CaMV 35S RNA promoter, a CaMV 19S RNA promoter, and a nopalin synthase gene promoter.

(Nucleic acid encoding g5151a gene or mutant gene thereof)

In one embodiment, the present invention provides a nucleic acid encoding a g5151a gene or a mutant gene thereof, in which a cDNA of the g5151a gene has a base sequence set forth in SEQ ID NO: 6, a cDNA of the mutant gene has a base sequence having 70% or more sequence identity with respect to the base sequence set forth in SEQ ID NO: 6, and a protein encoded by the mutant gene has an activity of converting PF1378C to PF1378B. The nucleic acid of the present embodiment may be a non-natural nucleic acid.
In the nucleic acid of the present embodiment, the g5151a gene and a mutant gene thereof are the same as described above. The mutant gene may have a base sequence having a sequence identity of 70% or more, 80% or more, 90% or more, 95% or more, 96% or more, 97% or more, 98% or more, or 99% or more with the base sequence set forth in SEQ ID NO: 6, and a protein encoded by the mutant gene may have an activity of converting PF1378C to PF1378B.
The nucleic acid of the present embodiment may be in the form of a nucleic acid fragment or may be in the form of a vector. That is, the nucleic acid of the present embodiment may be a vector including a nucleic acid encoding the g5151a gene or a mutant gene thereof. The vector is the same as described above.
The nucleic acid of the present embodiment preferably includes a selectable marker for selecting a transformant. The selectable marker is the same as described above.
The nucleic acid may have a transcription regulation signal or a translation regulation signal necessary for the expression of a gene. The transcriptional regulation signal or the translational regulation signal is the same as described above.

[Transformant]

(Transformant into which nucleic acid encoding g5141 gene or mutant gene thereof has been introduced)

In one embodiment, the present invention provides a transformant into which a nucleic acid encoding the g5141 gene or a mutant gene thereof has been introduced. The transformant of the present embodiment may be obtained by introducing a nucleic acid fragment into a host using homologous recombination technology, or may be obtained by introducing a vector into a host. Examples of the host include actinomycetes, Escherichia coli, Bacillus subtilis, a yeast, filamentous fungi, and plants. The nucleic acid is the same as described above and an appropriate nucleic acid may be appropriately selected depending on the host.
As a method for introducing a nucleic acid into a host, an appropriate method may be appropriately selected depending on the form of the nucleic acid and the host, and examples thereof include a conjugative transfer method, a transduction method using a phage, a calcium ion method, a lithium-ion method, an electroporation method, a PEG method, an Agrobacterium method, and a particle gun method.
The transformant of the present embodiment can be used, for example, as a storage medium (carrier) for the g5141 gene or a mutant gene thereof.
Alternatively, the protein encoded by the g5141 gene or a mutant gene thereof can be expressed in a cell of the transformant of the present embodiment. Therefore, the transformant of the present embodiment can be used for converting PF1378B to PF1378A.
Furthermore, in a case where the transformant of the present embodiment is capable of biosynthesizing PF1378B, the transformant of the present embodiment can be cultured to perform the biosynthesis of PF1378B and the conversion to PF1378A, thereby producing PF1378A. The method of culturing the transformant and the purification of the obtained PF1378A are the same as described above.

(Transformant into which nucleic acid encoding g5151a gene or mutant gene thereof has been introduced)

In one embodiment, the present invention provides a transformant into which a nucleic acid encoding the g5151a gene or a mutant gene thereof has been introduced. The transformant of the present embodiment may be obtained by introducing a nucleic acid fragment into a host using homologous recombination technology, or may be obtained by introducing a vector into a host. The host and the method for introducing the nucleic acid into the host are the same as described above.
The transformant of the present embodiment can be used, for example, as a storage medium (carrier) for the g5151a gene or a mutant gene thereof.
Alternatively, the protein encoded by the g5151a gene or a mutant gene thereof can be expressed in a cell of the

transformant of the present embodiment. Therefore, the transformant of the present embodiment can be used for converting PF1378C to PF1378B.

Furthermore, in a case where the transformant of the present embodiment is capable of biosynthesizing PF1378C, the transformant of the present embodiment can be cultured to perform the biosynthesis of PF1378C and the conversion to PF1378B.

Furthermore, in a case where the nucleic acid encoding the g5141 gene or a mutant gene thereof has been further introduced into the transformant of the present embodiment, PF1378B can be converted to PF1378A, thereby producing PF1378A. The method of culturing the transformant and the purification of the obtained PF1378A are the same as described above.

Examples

Next, the present invention will be described in more detail with reference to Examples, but the present invention is not limited to the following Examples.

[Experimental Example 1]

(Creation of non-homologous end joining function-deficient strain of A. aculeatinus PF1378)

A complete genome of an A. aculeatinus PF1378 was analyzed. From the whole genome analysis data, the presence of a homolog (g6418) of ku70, which is known to be involved in non-homologous end joining of Aspergillus oryzae, was confirmed. The base sequence of g6418 is shown in SEQ ID NO: 4. Accordingly, a knockout strain of g6418 was created by inserting a hygromycin-resistant gene into g6418.

A gene fragment including a 5' upstream partial fragment of g6418, an expression construct of the hygromycin-resistant gene, and a 3' downstream partial fragment of g6418 in this order was prepared. Subsequently, the obtained gene fragment was introduced into the A. aculeatinus PF1378 by a protoplast-PEG method. Subsequently, the cell was cultured in a Czapek-Dox agar medium including 25 μg/mL of hygromycin to select a target transformant. The obtained transformant in which the ku70 homolog was disrupted was named an A. aculeatinus PF1378 Δku70.

[Experimental Example 2]

(Creation of uracil-auxotrophic A. aculeatinus PF1378 Δku70)

From the whole genome analysis data of the A. aculeatinus PF1378, the presence of a homolog (g3123) of an enzyme gene pyrG constituting an uracil biosynthetic pathway of A. oryzae was confirmed. The base sequence of g3123 is shown in SEQ ID NO: 5. Accordingly, the pyrithiamine-resistant gene of A. oryzae as a selectable marker was inserted into g3123 to create an uracil-auxotrophic A. aculeatinus PF1378 Δku70.

A gene fragment including a 5' upstream partial fragment of g3123, an expression construct of the pyrithiamine-resistant gene, and a 3' downstream partial fragment of g3123 in this order was prepared. Subsequently, the obtained gene fragment was introduced into the A. aculeatinus PF1378 Δku70 by a protoplast-PEG method. Subsequently, a target transformant was selected on a Czapek-Dox agar medium including 1 μg/mL pyrithiamine hydrobromide, 5 mM uridine, and 10 mM uracil. The obtained pyrithiamine-resistant transformant, that is, the uracil-auxotrophic transformant was named an A. aculeatinus PF1378 Δku70 ΔpyrG.

[Experimental Example 3]

(Introduction of Cre/loxP marker recycling system into A. aculeatinus PF1378 Δku70 ΔpyrG)

A Cre/loxP marker recycling system using a pyrG gene derived from A. oryzae as a selectable marker was introduced into a site of the pyrithiamine-resistant gene of the A. aculeatinus PF1378 Δku70 ΔpyrG obtained in Experimental Example 2.

A gene fragment including a 5' upstream partial fragment of g3123, a lox66 sequence, an A. oryzae-derived pyrG gene, a lox71 sequence, an A. oryzae-derived xylanase gene promoter region, a Cre-encoding region, an A. oryzae-derived α-glucosidase gene terminator region, and a 3' downstream partial fragment of g3123 in this order was prepared. Subsequently, the obtained gene fragment was introduced into the A. aculeatinus PF1378 Δku70 ΔpyrG by a protoplast-PEG method. Subsequently, a target transformant was selected on a Czapek-Dox agar medium.

The strain in which the pyrithiamine-resistant gene of the obtained A. aculeatinus PF1378 Δku70 ΔpyrG was replaced with an A. oryzae-derived pyrG gene and a Cre/loxP marker recycling gene cassette was named an A.

aculeatinus PF1378 Δku70 Cre.

[Experimental Example 4]

(Removal of A. oryzae-derived pyrG gene from A. aculeatinus PF1378 Δku70 Cre)

The A. aculeatinus PF1378 Δku70 Cre created in Experimental Example 3 was cultured in a Czapek-Dox medium including 5 mM uridine, 10 mM uracil, and 1 mg/mL 5-fluoroorotic acid, and having 2% xylose as a carbon source, and a Cre/loxP marker recycling system was induced to select a strain from which the A. oryzae-derived pyrG gene had been removed. The strain from which the obtained A. oryzae-derived pyrG gene had been removed was named an A. aculeatinus PF1378 Δku70 Cre [pyrG-].

[Experimental Example 5]

(Removal of g5141 gene from A. aculeatinus PF1378 Δku70 Cre [pyrG-])

The presence of a g5141 gene was confirmed from the whole genome analysis data of the A. aculeatinus PF1378. The base sequence of a cDNA of the g5141 gene is shown in SEQ ID NO: 1. Furthermore, the amino acid sequence of the protein encoded by the g5141 gene is shown in SEQ ID NO: 2. In addition, the base sequence of a genomic DNA of the g5141 gene is shown in SEQ ID NO: 3.

Subsequently, a g3123 gene, which is the pyrG gene of A. aculeatinus PF1378, was inserted into the g5141 gene locus to create a knockout strain of g5141.

A gene fragment including a 5' upstream partial fragment of g5141, a g3123 region which is the pyrG gene of PF1378, and a 3' downstream partial fragment of g5141 in this order was prepared. Subsequently, the obtained gene fragment was introduced into the A. aculeatinus PF1378 Δku70 Cre [pyrG-] by a protoplast-PEG method. Subsequently, a target transformant was selected on a Czapek-Dox agar medium. The obtained g5141-deficient strain was named an A. aculeatinus PF1378 Δku70 Cre Δg5141.

[Experimental Example 6]

(Removal of g5151a gene from A. aculeatinus PF1378 Δku70 Cre [pyrG-])

The presence of a g5151a gene was confirmed from the whole genome analysis data of the A. aculeatinus PF1378. The base sequence of a cDNA of the g5151a gene is shown in SEQ ID NO: 6. Furthermore, the amino acid sequence of a protein encoded by the g5151a gene is shown in SEQ ID NO: 7. In addition, the base sequence of a genomic DNA of the g5151a gene is shown in SEQ ID NO: 8.

Subsequently, a g3123 gene, which is the pyrG gene of A. aculeatinus PF1378, was inserted into the g5151a gene locus to create a knockout strain of g5151a.

A gene fragment including a 5' upstream partial fragment of g5151a, a g3123 region which is the pyrG gene of PF1378, and a 3' downstream partial fragment of g5151a in this order was prepared. Subsequently, the obtained gene fragment was introduced into the A. aculeatinus PF1378 Δku70 Cre [pyrG-] by a protoplast-PEG method. Subsequently, a target transformant was selected on a Czapek-Dox agar medium. The obtained g5151a-deficient strain was named an A. aculeatinus PF1378 Δku70 Cre Δg5151a.

[Experimental Example 7]

(Examination of production ability of PF1378A and PF1378B of A. aculeatinus PF1378 Δku70 Cre Δg5141)

The production ability of PF1378A and PF1378B in the A. aculeatinus PF1378 Δku70 Cre Δg5141 was examined.

Each of the A. aculeatinus PF1378 and the A. aculeatinus PF1378 Δku70 Cre Δg5141 was cultured on a potato dextrose agar medium, and spores were collected. Subsequently, the spores suspended in a 0.01% aqueous Tween20 solution were diluted to a concentration of $4 \times 10^5$ spores/mL to obtain a spore suspension.

Brown rice (Akitakomachi) which had been soaked in water overnight was sterilized by an autoclave at 121°C for 20 minutes to prepare a rice medium. 5 g of the prepared rice medium was charged into a bioreactor tube (TPP(R) TubeSpin 50 mL) and 500 μL of the spore suspension was inoculated thereto. Subsequently, the rice medium inoculated with the spore suspension was statically cultured at 25°C, and 7 days later, 10 mL of a 67% aqueous acetone solution was added thereto. After the addition, the mixture was thoroughly stirred with a shaker for 1 hour and centrifuged at 4°C and 10,000 × g for 60 minutes. The obtained supernatant was subjected to HPLC to quantify PF1378A and PF1378B. The test

was carried out with n = 5.

The quantitative results of PF1378A are shown in Table 1 below and the quantitative results of PF1378B are shown in Table 2 below. As a result, in the A. aculeatinus PF1378, the production of both PF1378A and PF1378B was detected. On the other hand, in the A. aculeatinus PF1378 Δku70 Cre Δg5141, the production of PF1378B was detected, but the production of PF1378A was not detected. Therefore, it was confirmed that the protein encoded by the g5141 gene is involved in the conversion from PF1378B to PF1378A. That is, it was revealed that a transformant in which g5141 gene is introduced into a microorganism that does not possess g5141 gene and produces PF1378B, or a transformant in which g5141 gene is introduced into a microorganism to which PF1378B can be applied externally, can produce PF1378A.

[Table 1]

| Concentration of PF1378A | | | | | | |
|---|---|---|---|---|---|---|
| Strain | PF1378A (μg/mL) | | | | | |
| | 1 | 2 | 3 | 4 | 5 | Average |
| A. aculeatinus PF1378 Δku70 Cre Δg5141 | 0.0 | 0.0 | 0.0 | 0.0 | 0.0 | 0.0 |
| A. aculeatinus PF1378 | 9.6 | 9.3 | 9.1 | 9.3 | 9.1 | 9.3 |

[Table 2]

| Concentration of PF1378B | | | | | | |
|---|---|---|---|---|---|---|
| Strain | PF1378B (μg/mL) | | | | | |
| | 1 | 2 | 3 | 4 | 5 | Average |
| A. aculeatinus PF1378 Δku70 Cre Δg5141 | 68.3 | 48.8 | 61.1 | 58.3 | 0.0 | 47.3 |
| A. aculeatinus PF1378 | 57.3 | 60.7 | 55.2 | 57.7 | 65.6 | 59.3 |

[Experimental Example 8]

(Examination of production ability of PF1378A, PF1378B, and PF1378C of A. aculeatinus PF1378 Δku70 Cre Δg5151a)

The production ability of PF1378A, PF1378B, and PF1378C in the A. aculeatinus PF1378 Δku70 Cre Δg5151a was examined.

Each of the A. aculeatinus PF1378 and the A. aculeatinus PF1378 Δku70 Cre Δg5151a was cultured on a potato dextrose agar medium, and spores were collected. Subsequently, the spores suspended in a 0.01% aqueous Tween20 solution were diluted to a concentration of $4 \times 10^5$ spores/mL to obtain a spore suspension.

Barley (barley produced in Saga Prefecture) which had been soaked in water for 2 hours was sterilized by an autoclave at 121°C for 20 minutes to prepare a barley medium. 5 g of the prepared barley medium was charged into a bioreactor tube (TPP(R) TubeSpin 50 mL) and 500 μL of the spore suspension was inoculated thereto. Subsequently, the barley medium inoculated with the spore suspension was statically cultured at 25°C, and 7 days later, 10 mL of a 67% aqueous acetone solution was added thereto. After the addition, the mixture was thoroughly stirred with a shaker for 1 hour and centrifuged at 4°C and 10,000 $\times$ g for 60 minutes. The obtained supernatant was dried and solidified with a centrifugal concentrator, and the obtained product dissolved in the same amount of methanol was used as a sample to subject PF1378A, PF1378B, and PF1378C to measurements by LCMS.

The quantitative results of PF1378A, PF1378B, and PF1378C are shown in Table 3 below. In Table 3, "N. D." indicates that the value is below a detection limit. The peak surface area of m/z 374 was defined as a production amount of PF1378A, the peak surface area of m/z 360 was defined as a production amount of PF1378B, the peak surface area of m/z 346 was defined as a production amount of PF1378C, and the value was shown by a relative value with respect to the production amount of PF1378A in the A. aculeatinus PF1378 strain set to 100 and the production amount of PF1378B set to 100. In addition, the production amount of PF1378C is shown by a relative value with respect to the production amount of PF1378B in the A. aculeatinus PF1378 strain set as 100.

As a result, in the A. aculeatinus PF1378, the production of both PF1378A and PF1378B was detected. On the other hand, in the A. aculeatinus PF1378 Δku70 Cre Δg5151a, the production of PF1378C was detected, but the production of PF1378A and PF1378B was not detected. Therefore, it was confirmed that the protein encoded by the g5151a gene is involved in the conversion from PF1378C to PF1378B. That is, it was revealed that a transformant in which g5151a gene is introduced into a microorganism that does not possess g5151a gene and produces PF1378C, or a

transformant in which g5151a gene is introduced into a microorganism to which PF1378C can be applied externally, can produce PF1378B.

[Table 3]

| Relative concentration of PF1378A, PF1378B, and PF1378C | | | |
|---|---|---|---|
| | PF1378A | PF1378B | PF1378C |
| A. aculeatinus PF1378 Δku70 Cre Δg5151a | N.D. | N.D. | 48 |
| A. aculeatinus PF1378 | 100 | 100 | N.D. |

[Experimental Example 9]

(Removal of g5141 gene from A. aculeatinus PF1378 Δku70 Cre [pyrG-], removal of pyrG gene, and complementary introduction of g5141 gene)

A g3123 gene, which is the pyrG gene of A. aculeatinus PF1378, was inserted into the g5141 gene locus of A. aculeatinus PF1378, to create a knockout strain of g5141. Here, the pyrG gene was designed so that it could be removed after the creation of the knockout strain. After creating a knockout strain, the pyrG gene was removed, and finally, g5141 gene was complementary introduced in a form that g5141 is constantly expressed using the pyrG gene as a selectable marker.

A gene fragment including a 5' upstream partial fragment of g5141, a lox66 sequence, a g3123 gene which is the pyrG gene of PF1378, a lox71 sequence, and a 3' downstream partial fragment of g5141 in this order was prepared. Subsequently, the obtained gene fragment was introduced into the A. aculeatinus PF1378 Δku70 Cre [pyrG-] by a protoplast-PEG method. Subsequently, a target transformant was selected on a Czapek-Dox agar medium. The obtained strain in which g5141 gene was deficient and pyrG gene can be removed was named an A. aculeatinus PF1378 Δku70 Cre Δg5141::lox66::pyrG::lox71.

Subsequently, the A. aculeatinus PF1378 Δku70 Cre Δg5141::lox66::pyrG::lox71 was cultured in a Czapek-Dox medium including 5 mM uridine, 10 mM uracil, and 1 mg/mL 5-fluoroorotic acid, and having 2% xylose as a carbon source, and a Cre/loxP marker recycling system was induced to select a strain from which the pyrG gene had been removed. The strain from which the pyrG gene had been removed was named an A. aculeatinus PF1378 Δku70 Cre Δg5141 [pyrG-].

The presence of a g4093, which is a homolog of tef1, which is a translation elongation factor 1-α gene of A. oryzae, was confirmed from the whole genome analysis data of the A. aculeatinus PF1378. The nucleotide sequence of the promoter of g4093, the length of which is 1012 bp, is shown in SEQ ID NO: 9. A strain was constructed by introducing g5141 into A. aculeatinus PF1378 Δku70 Cre Δg5141 [pyrG-] so that g5141 is expressed by a promoter of g4093. A gene fragment including a 5' upstream partial fragment of g5141, a g3123 gene which is the pyrG gene of PF1378, a promoter region of g4093, a coding region of g5141 which is shown in SEQ ID NO: 3, a 3' downstream partial fragment of g5141 in this order was prepared. Subsequently, the obtained gene fragment was introduced into the A. aculeatinus PF1378 Δku70 Cre Δg5141 [pyrG-] by a protoplast-PEG method. Subsequently, a target transformant was selected on a Czapek-Dox agar medium. The obtained strain in which g5141 gene is introduced so that g5141 gene is expressed by a promoter of g4093 was named an A. aculeatinus PF1378 Δku70 Cre Δg5141::pyrG::Pg4093::g5141.

[Experimental Example 10]

(Examination of production ability of PF1378A and PF1378B of A. aculeatinus PF1378 Δku70 Cre Δg5141::pyrG::Pg4093::g5141)

The production ability of PF1378A and PF1378B in the strain obtained in the Experimental Example 9, in which g5141 gene is introduced into A. aculeatinus PF1378 Δku70 Cre Δg5141 [pyrG-] was examined.

Barley (barley produced in Saga Prefecture) which had been soaked in water for 2 hours was sterilized by an autoclave at 121°C for 20 minutes to prepare a barley medium. 5 g of the prepared barley medium was charged into a bioreactor tube (TPP(R) TubeSpin 50 mL) and 500 μL of the spore suspension was inoculated thereto. Subsequently, the barley medium inoculated with the spore suspension was statically cultured at 25°C, and 7 days later, 10 mL of a 67% aqueous acetone solution was added thereto. After the addition, the mixture was thoroughly stirred with a shaker for 1 hour and centrifuged at 4°C and 10,000 × g for 60 minutes. The obtained supernatant was used as a sample to subject PF1378A and PF1378B to measurements by LCMS. The test was carried out with n = 2.

The quantitative results of PF1378A are shown in Table 4 below and the quantitative results of PF1378B are shown in Table 5 below. As a result, in the A. aculeatinus PF1378 Δku70 Cre Δg5141::pyrG::Pg4093::g5141, the

production of both PF1378A and PF1378B was detected. On the other hand, as shown in the Experimental Example 7, A. aculeatinus PF1378 Δku70 Cre Δg5141 do not produce PF1378A, therefore, this result further supports that the protein encoded by the g5141 gene is involved in the conversion from PF1378B to PF1378A. That is, it was further supported that a transformant in which g5141 gene is introduced into a microorganism that does not possess g5141 gene and produces PF1378B, or a transformant in which g5141 gene is introduced into a microorganism to which PF1378B can be applied externally, can produce PF1378A.

[Table 4]

| Concentration of PF1378A | | | |
|---|---|---|---|
| Strain | PF1378A (mg/L) | | |
| | 1 | 2 | Average |
| A. aculeatinus PF1378 Δku70 Cre Δg5141::pyrG::Pg4093::g5141 | 11.0 | 11.0 | 11.0 |

[Table 5]

| Concentration of PF1378B | | | |
|---|---|---|---|
| Strain | PF1378B (mg/L) | | |
| | 1 | 2 | Average |
| A. aculeatinus PF1378 Δku70 Cre Δg5141::pyrG::Pg4093::g5141 | 29.2 | 29.8 | 29.5 |

INDUSTRIAL APPLICABILITY

According to the present invention, it is possible to provide production technology for PF1378A.

## Claims

1. A production method for PF1378A, the method comprising:
a step (a) of bringing one or more proteins selected from the group consisting of the following (1) to (4) into contact with PF1378B to obtain PF1378A,

    (1) a protein consisting of an amino acid sequence set forth in SEQ ID NO: 2,
    (2) a protein consisting of an amino acid sequence having a sequence identity of 70% or more with respect to the amino acid sequence set forth in SEQ ID NO: 2 and has an activity of converting PF1378B to PF1378A,
    (3) a protein encoded by a base sequence set forth in SEQ ID NO: 1, and
    (4) a protein encoded by a base sequence having a sequence identity of 70% or more with respect to the base sequence set forth in SEQ ID NO: 1 and has an activity of converting PF1378B to PF1378A.

2. The production method according to Claim 1,
wherein the step (a) is carried out in a cell of a microorganism.

3. The production method according to Claim 1 or 2, further comprising, before the step (a):

    a step (b) of bringing one or more proteins selected from the group consisting of the following (5) to (8) into contact with PF1378C to obtain PF1378B,
    (5) a protein consisting of an amino acid sequence set forth in SEQ ID NO: 7,
    (6) a protein consisting of an amino acid sequence having a sequence identity of 70% or more with respect to the amino acid sequence set forth in SEQ ID NO: 7 and has an activity of converting PF1378C to PF1378B,
    (7) a protein encoded by a base sequence set forth in SEQ ID NO: 6, and
    (8) a protein encoded by a base sequence having a sequence identity of 70% or more with respect to the base sequence set forth in SEQ ID NO: 6 and has an activity of converting PF1378C to PF1378B.

4. The production method according to Claim 3,
wherein the step (b) is carried out in a cell of a microorganism.

5. A protein consisting of an amino acid sequence set forth in SEQ ID NO: 2, or consisting of an amino acid sequence having a sequence identity of 70% or more with respect to the amino acid sequence set forth in SEQ ID NO: 2 and has an activity of converting PF1378B to PF1378A.

6. A protein consisting of an amino acid sequence set forth in SEQ ID NO: 7, or consisting of an amino acid sequence having a sequence identity of 70% or more with respect to the amino acid sequence set forth in SEQ ID NO: 7 and has an activity of converting PF1378C to PF1378B.

7. A nucleic acid encoding the protein according to Claim 5.

8. The nucleic acid according to Claim 7,
wherein the nucleic acid consists of a base sequence set forth in SEQ ID NO: 1; or consists of a base sequence having a sequence identity of 70% or more with respect to the base sequence set forth in SEQ ID NO: 1 and encodes a protein having an activity of converting PF1378B to PF1378A.

9. A nucleic acid encoding the protein according to Claim 6.

10. The nucleic acid according to Claim 9,
wherein the nucleic acid consists of a base sequence set forth in SEQ ID NO: 6; or consists of a base sequence having a sequence identity of 70% or more with respect to the base sequence set forth in SEQ ID NO: 6 and encodes a protein having an activity of converting PF1378C to PF1378B.

11. A transformant into which the nucleic acid according to Claim 7 or 8 has been introduced.

12. A transformant into which the nucleic acid according to Claim 9 or 10 has been introduced.

**INTERNATIONAL SEARCH REPORT**

International application No. **PCT/JP2024/018666**

**A. CLASSIFICATION OF SUBJECT MATTER**

***C12N 15/31***(2006.01)i; ***C12N 1/15***(2006.01)i; ***C12N 1/19***(2006.01)i; ***C12N 1/21***(2006.01)i; ***C12P 17/12***(2006.01)i
FI: C12N15/31; C12N1/15; C12N1/19; C12N1/21; C12P17/12 ZNA

According to International Patent Classification (IPC) or to both national classification and IPC

**B. FIELDS SEARCHED**

Minimum documentation searched (classification system followed by classification symbols)

C12N15/31; C12N1/15; C12N1/19; C12N1/21; C12P17/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq; UniProt/GeneSeq; SwissProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X | Database GenBank [online], Accession No. RAK81182, 22 June 2018 [retrieved on 11 June 2024], Internet: <URL : https://www.ncbi.nlm.nih.gov/protein/RAK81182.1>, in particular, amino acid sequence | 6, 9-10, 12 |
| A | WO 2020/241702 A1 (MEIJI SEIKA PHARMA CO., LTD.) 03 December 2020 (2020-12-03) entire text, all drawings | 1-12 |
| A | INGAVAT, Nattha et al. Asperaculin A, a Sesquiterpenoid from a Marine-Derived Fungus, Aspergillus aculeatus. Journal of Natural Products, 13 June 2011, 74, pp. 1650-1652, in particular, p. 1650, left column, 1st paragraph | 1-12 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 July 2024** | **16 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/018666**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/018666**

| Patent document cited in search report | Publication date (day/month/year) | Patent family member(s) | Publication date (day/month/year) |
|---|---|---|---|
| WO 2020/241702 A1 | 03 December 2020 | US 2022/0225618 A1<br>entire text, all drawings<br>EP 3977856 A1<br>CN 113891655 A<br>KR 10-2022-0012879 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023084745 A
- WO 2008156165 A
- WO 2010071218 A
- WO 2010071219 A


### Non-patent literature cited in the description


- *CHEMICAL ABSTRACTS*, 199784-50-4
- **BANKS R. M. et al.** Novel anthelmintic metabolites from an Aspergillus species; the aspergillimides. *The Journal of Antibiotics*, 1997, vol. 50 (10), 840-846
- *CHEMICAL ABSTRACTS*, 195966-93-9
- *CHEMICAL ABSTRACTS*, 227598-73-4